# EUROPEAN PATENT SPECIFICATION

(11) **EP 1 652 501 B1**
(45) Date of publication and mention of the grant of the patent: **28.10.2009**
(21) Application number: 04748117.1
(22) Date of filing: 30.07.2004
(51) Int. Cl.: A61F 13/56

(54) **DISPOSABLE DIAPER**
EINWEGWINDEL
COUCHE JETABLE

(30) Priority: 30.07.2003 JP 2003204005; 16.07.2004 JP 2004210084
(43) Date of publication of application: 03.05.2006
(73) Proprietor: UNI-CHARM CO., LTD., Shikikucho-shi, Ehime 799-0111 (JP)
(72) Inventor: MINATO, Hironao, Techn. Center,UNI-CHARM CO. LTD., Kanonji-shi, Kagawa 769-1602 (JP)
(74) Representative: Murgatroyd, Susan Elizabeth
(86) International application number: PCT/JP2004/010956
(87) International publication number: WO 2005/011550

(56) References cited:
- EP-A- 0 934 735
- WO-A-96/10979
- WO-A-99/09926
- DE-A1- 3 245 196
- JP-A- 10 165 439
- JP-A- 2000 509 672
- US-A- 4 591 523
- US-A1- 2003 028 165

## Description

### TECHNICAL FIELD OF THE INVENTION

The present invention relates to a disposable diaper adapted for absorption and containment of bodily discharges.

There has already been proposed a disposable diaper comprising a liquid-pervious topsheet, a liquid-impervious backsheet and a liquid-absorbent core interposed between these two sheets so as to define, in a longitudinal direction, a front waist region, a rear waist region and a crotch region extending between these waist regions. The diaper is contoured by longitudinally opposite ends extending in a transverse direction outside longitudinally opposite ends of the core and transversely opposite side edges extending in the longitudinal direction outside transversely opposite ends of the core. The front waist region is provided with a pair of first wings extending outward from respective side edges of the front waist region in the transverse direction and the rear waist region is provided with a pair of second wings extending outward from the respective side edges of the rear waist region in the transverse direction (See Patent Document 1).

In the diaper disclosed in Patent Document 1, the wings of the rear waist region is provided with a pair of tape fasteners and the front waist region is provided on its outer surface with a target tape on which the tape fasteners are releasably anchored. Each of the second wings extending from the rear waist region comprises an inner layer sheet and an outer layer sheet both of which are elastically stretchable. These inner layer sheet and outer layer sheet may be made of an elastically stretchable fibrous nonwoven fabric, elastomer film or rubber film. The inner layer sheet and the outer layer sheet are overlapped and bonded together through the intermediary of elastic material having a rubber-like elasticity and a stretch stress higher than those of the inner layer sheet and the outer layer sheet. Each of the tape fastener has a fixed side portion which is permanently bonded to the outer end of the associated second wing and a free portion extending outward from the fixed side portion in the transverse direction. A hook member of a mechanical fastener is attached to the free portion of the tape fastener and a loop member of the mechanical fastener is attached to outer surface of the target tape.

To put this diaper on the wearer's body, the second wings of the rear waist region are placed upon the outer side of the front waist region and then the free portions of the respective tape fasteners are anchored on the outer surface of the target tape to connect the front and rear waist regions with each other. Upon connection of the front and rear waist regions, the diaper is formed with a waist-hole and a pair of leg-holes. This diaper is characterized in that the inner layer sheet and the outer layer sheet constituting the second wings are bonded together in a non-stretched state through the intermediary of the elastic material, so there is no possibility that the inner layer sheet and the outer layer sheet might be formed with gathers which might create a feeling of discomfort against the wearer.
Patent Document 1: Japanese Unexamined Patent Application Publication No. 1998-328237
US 2003/028165 A1 discloses a multilayer laminate web wherein at least a central layer is apertured. One example of laminate web is a film/nonwoven laminate useful for a stretch side panel of a disposable diaper. The laminate web comprises a first web; a second web joined to the first web at a plurality of discrete bond sites; and a third material disposed between at least a portion of the first and second non-wovens. The third material is apertured in regions adjacent the bond sites, such that the first and second non-woven webs are joined through the apertures.
DE 3245196 A1 discloses a process for producing a porous material web, in particular of plastic-coated paper or non-woven fabric, in which a porosity such that the material permeable to gas and water vapour but substantially liquid-tight is produced by an electrical micro-perforation. The plastic-coated nonwoven fabrics are particularly well sited for use in the hygiene sector.
WO 96/10979 A discloses a coversheet for covering an absorbent body of an absorbent sanitary article. The coversheet is elastic in at least one direction and has perforations which extend therethrough. It comprises: an upper layer intended to face outwardly of the absorbent body and comprising a non-woven fibrous material; an intermediate layer comprising an elastic film; and a lower layer intended to face inwardly towards the absorbent body and comprising a non-woven fibrous material. The upper and lower layers are connected to the intermediate layer substantially only around the perimeters of the perforations.
WO 99/09926 A discloses a method for aperturing a laminate for an elasticized side panel of a disposable diaper. The laminate has a topsheet and a backsheet each formed of a non-woven web of thermoplastic fibers, and an elastic member between the sheets. The elasticized side panel contains apertures which extend completely through the topsheet, the backsheet and the elastic member.
EP-A-0934735 disclose a diaper having an apertured backsheet for breathability. At least one of the breathable layers of the backsheet comprises a resilient, three dimensional web which consists of a liquid impervious polymeric film having apertures. The apertures form capillaries which are not perpendicular to the plane of the film.
US-A-4591523 discloses an apertured, macroscopically expanded, resilient three-dimensional polymeric web for use as breatheable barriers for diaper. The web comprises a multiplicity of debossments each originating as an aperture in a first surface of the web and having continuously interconnected side wall extending in the direction of second, remotely located parallel surface of the web. The side wall of each debossment terminates to form an end wall in the second surface of the web. The end wall includes a multiplicity of apertures, each of said apertures being sized and shaped to independently support an aqueous fluid meniscus.

### DISCLOSURE OF THE INVENTION

### PROBLEM TO BE SOLVED BY THE INVENTION

The diaper disclosed in Patent Document 1 uses an elastically stretchable fibrous non-woven fabric, elastomer film or rubber film as stock materials for the inner layer sheet and the outer layer sheet constituting the second wings of the rear waist region. Disadvantageously, the wearer may experience uncomfortable tackiness and sliminess peculiar to elastomeric materials as these sheets formed from the elastomer or rubber film and the elastically stretchable fibrous nonwoven fabric containing an elastomeric materials as these sheets formed from the elastomer or rubber film and the elastically stretchable fibrous nonwoven fabric containing an elastomeric material come in contact with the wearer's skin. In addition, these sheets may stick to the wearer's skin since these sheets exhibit a relatively high frictional force and such factor also may deteriorate a feeling to wear the diaper. When the elastomer film or the rubber film is used as stock material for the inner layer sheet and the outer layer sheet, a water vapor permeability of the second wings extending from the rear waist region will be significantly deteriorated and it will be impossible to let water vapor generated due to evaporation of sweat out from the diaper through the second wings. Consequently, a stuffiness generated within the diaper can not be eliminated.

In view of the problems as have been described above, it is an object of the present invention to provide a disposable diaper improved so as to give the wearer no uncomfortable feeling to wear this diaper, on one hand, and to allow water vapor generated as a result of sweat evaporation to be let out from the diaper through the wings, on the other hand.

### MEASURE TO SOLVE THE PROBLEM

The object set forth above is achieved, according to the present invention, by a disposable diaper comprising a front waist region; a rear waist region a crotch region extending between these two waist regions; a pair of first wings extending outward from transversely opposite side edge portions of said front waist region in a transverse direction; a pair of second wings extending outward from transversely opposite side edge portions of said rear waist region in the transverse direction; of said first and second wings, at least said second wings are formed from an elastically stretchable composite sheet which comprises, in turn, a breathable stretchy plastic film formed with a plurality of air passages extending through said plastic film each having a first opening facing the wearer's skin and a second opening facing away from the wearer's skin; a pair of breathable, heat-sealable fibrous non-woven fabric layers partially bonded to both surfaces of said plastic film at heat-sealed spots, said diaper being characterized in that: said fibrous non-woven fabric layers are formed with a plurality of gathers rising and falling along surfaces of said composite sheet; the number of said air passages formed through said plastic film is in a range of 10 - 80/cm² of said plastic film and a total area ratio of said first openings is in a range of 10 - 20% of the plastic film; inside said air passage, said fibrous nonwoven fabric layers are bonded together at said heat-sealed spots and the number of said air passages inside which said fibrous non-woven fabric layers are bonded to each other is in a range of 1.5 - 10%; and each of said wings formed from said elastically stretchable composite sheet presents a transverse stretch stress of 1.5 - 7.0 N as said wing is stretched in the transverse direction to a length dimension in the transverse direction corresponding to 120% of its non-stretched transverse length dimension set as 100% and presents a transverse stretch stress of 3.0 - 10.0 N as said wing is stretched to a length dimension in the transverse direction corresponding to 150% of said non-stretched length dimension.

The wings are free from uncomfortable tackiness and sliminess peculiar to rubber-based materials although the wings are elastically stretchable as a whole, since one of the fibrous non-woven fabric layers comes in contact with the wearer's skin. Furthermore, water vapor generated due to sweat evaporation can be let out from the diaper through the air passages.

The diaper according to the present invention includes the following preferred embodiments.
The plastic film is formed from an elastically stretchable thermoplastic elastomer film and a pair of polyolefin-based thermoplastic synthetic resin film layers bonded to both surfaces of the elastomer film, and the fibrous non-woven fabric is formed from polyolefin-based thermoplastic synthetic resin fibers.

The second opening has an area smaller than that of the first opening so that the air passage is tapered from the first opening toward the second opening. A surface tension is higher in the vicinity of the second opening than in the vicinity of the first opening.

This diaper allows water vapor generated due to evaporation of the sweat having transferred to the heat-sealed spots to be let out from the diaper.

The fibrous non-woven fabric layers maintain a fibrous state at the heat-sealed spots and have a fibrous density higher at the heat-sealed spots than in the remaining region except the heat-sealed spots.
A capillary phenomenon occurs more significantly at the heat-sealed spots than in the remaining region and an amount of sweat having permeated a remaining region of the non-woven fabric layer rapidly transfers toward the heat-sealed spots.

The plastic film has a thickness of 0.3 - 1 mm, and the fibrous non-woven fabric layers each, has a basic weight of 20 - 60 g/m² glint a thickness of 0.1 - 0.5 mm and a density of 0.04 - 0.6 g/cm³.

### EFFECT OF THE INVENTION

The disposable diaper according to the present invention is primarily characterized in that at least the second wings of the first and second wings are formed from the elastically stretchable composite sheet comprising the breathable and stretchy plastic film and the breathable heat-sealable fibrous nonwoven fabric layers partially bonded to both surfaces of the elastic film wherein one of the fibrous nonwoven fabric layers comes in contact with the wearer's skin as the diaper is put on. Such features are effective to eliminate the apprehension that the wings might stick to the wearer's skin and the wearer might experience uncomfortable feeling to wear the diaper although the wings are elastically stretchable as a whole. On the other important aspect of the present invention, the stretchy plastic film is formed with a plurality of the air passages allowing water vapor generated due to sweat evaporation to be let out from the diaper through these air passages and thereby allowing stuffiness possibly occurring within the diaper to be prevented. Furthermore, the elasticity of the wings may be utilized to tighten the diaper around the wearer's waist and thereby may prevent the diaper once put on the wearer's body from slipping down from its proper position.

In the diaper wherein the fibrous nonwoven fabric made of polyolefin-based thermoplastic synthetic resin fiber is used as the constituent layers of the composite sheet, the wings are free from uncomfortable tackiness and sliminess peculiar to the rubber-based material and there is no anxiety that the wearer might experience uncomfortable feeling as the wings come in contact with skin.

In the diaper wherein each of the air passages formed through the stretchy plastic film constituting the composite sheet is tapered from the first opening toward the second opening, a surface tension acting on the air passage is higher in the vicinity of the second opening than in the vicinity of the first opening. Consequently, sweat having flowed into the first opening can be easily transferred toward the second opening, i.e., toward the skin non-contacting side of the wing under the surface tension. In this way, wetness on this side of the wing can be rapidly eliminated.

In the diaper wherein the fibrous nonwoven fabric layers constituting the composite sheet are bonded to each other inside the air passage at the heat-sealed spots, water vapor generated due to evaporation of sweat having moved to the heat-sealed spots can be let out from the diaper through these air passages and the amount of sweat having permeated the nonwoven fabric layer can be rapidly dried.

In the diaper wherein the nonwoven fabric layers have fiber densities higher in the heat-sealed spots than in the respective remaining regions, a capillary phenomenon occurs more significantly in the heat-sealed spots than in the remaining regions. Such significant capillary phenomenon allows any amount of sweat having permeated the remaining region of the nonwoven fabric layer to be rapidly transferred to the heat-sealed spots and thereby allows the remaining region to be rapidly dried. In this way, uncomfortable tackiness and/or sliminess due to sweat which would otherwise stay on the remaining region of the nonwoven fabric layer destined to contact with the wearer's skin can be effectively eliminated.

### BRIEF DESCRIPTION OF THE DRAWINGS

[FIG.1] Fig. 1 is a partially cutaway perspective view showing an open-type diaper, one of embodiments of the invention.
[FIG. 2] Fig. 2 is a sectional view taken along the line II-II in Fig. 1.
[FIG. 3] Fig. 3 is a sectional view taken along the line III-III in Fig. 1.
[FIG. 4] Fig. 4 is a sectional view taken along the line IV-IV in Fig. 1.
[FIG. 5] Fig. 5 is a partially cutaway perspective view of a composite sheet.
[FIG. 6] Fig. 6 is a sectional view taken along the line VI-VI in Fig. 5.
[FIG. 7] Fig. 7 is a perspective view showing the diaper as put on the wearer's body.
[FIG. 8] Fig. 8 is a perspective view showing a pull-on diaper as another embodiment of the invention.
[FIG. 9] Fig. 9 is a sectional view taken along the line IX-IX in Fig. 8.
[FIG. 10] Fig. 10 is a developed and partially cutaway view showing the diaper of Fig. 8 with the front and rear waist regions having been disconnected from each other.
[FIG. 11] Fig. 11 is an end view showing the composite sheet forming the first and second wings.

### IDENTIFICATION OF REFERENCE NUMERALS USED IN THE DRAWINGS

- 1A: open-type diaper (disposable diaper)
- 1B: pull-on diaper (disposable diaper)
- 2: liquid-pervious topsheet
- 3: liquid-impervious backsheet
- 4: liquid-absorbent core
- 4a: longitudinally opposite ends
- 4b: transversely opposite side edges
- 8: front waist region
- 9: crotch region
- 10: rear waist region
- 11: longitudinally opposite end portions
- 12: transversely opposite side edge portions
- 13: first wings
- 13a: transversely inner end portions
- 13b: transversely outer end portions
- 14: first wings
- 14a: transversely inner end portions
- 14b: transversely outer end portions
- 18: elastically stretchable composite sheet
- 25: breathable and stretchable plastic film
- 26: breathable and heat-sealing fibrous nonwoven fabric layers
- 27: heat-sealed spots
- 28: gathers
- 29: thermoplastic elastomer film layer
- 30: thermoplastic synthetic resin film layer
- 31: air passages
- 32: first opening
- 33: second opening
- 34: tubular wall
- 35: heat-sealed spots

### DESCRIPTION OF THE BEST MODE FOR WORKING OF THE INVENTION

Details of a disposable diaper according to the present invention will be more fully understood from the description of open-type and pull-on diapers as typical embodiments given hereunder with reference to the accompanying drawings.

### EMBODIMENT 1

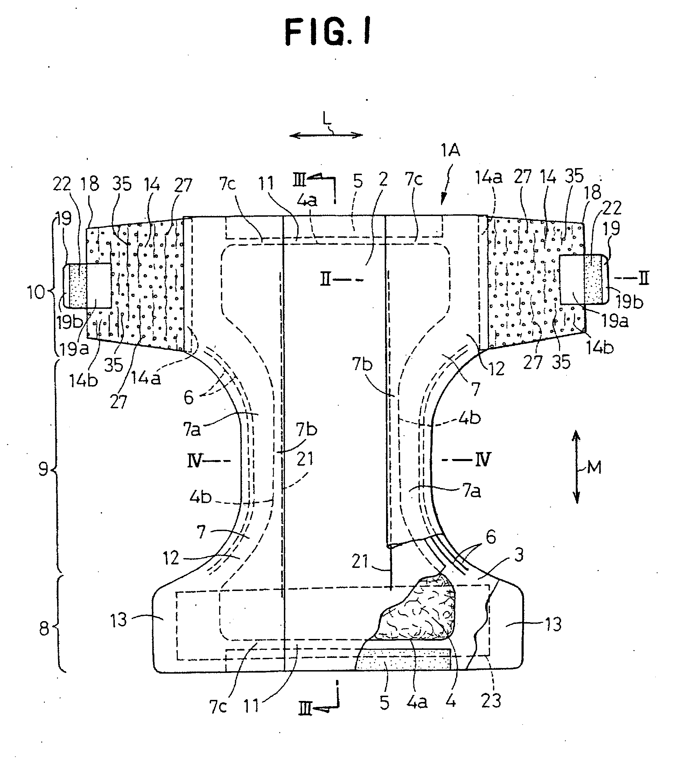

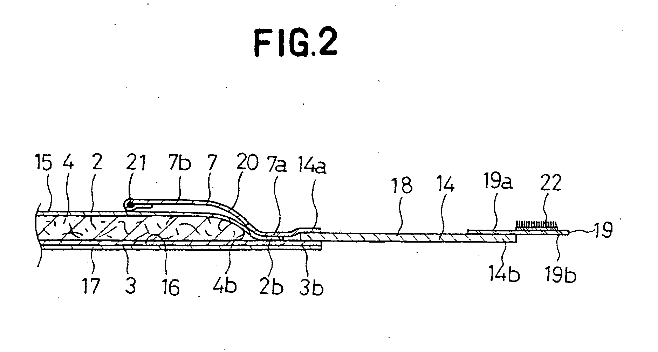

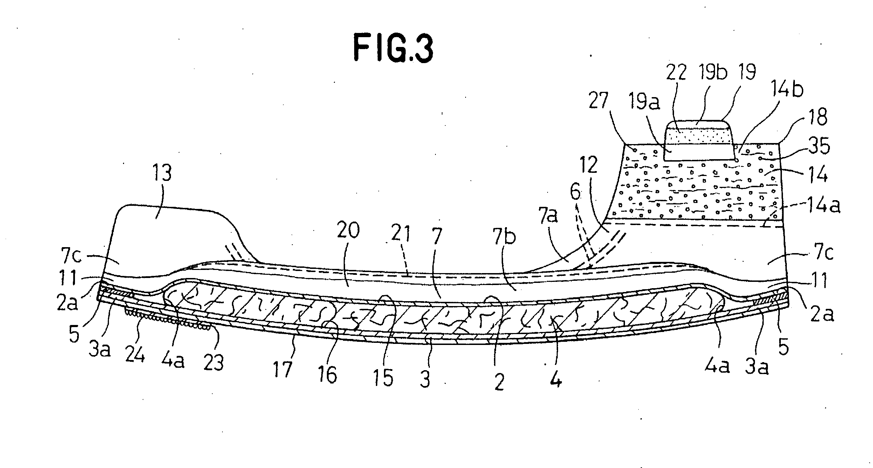

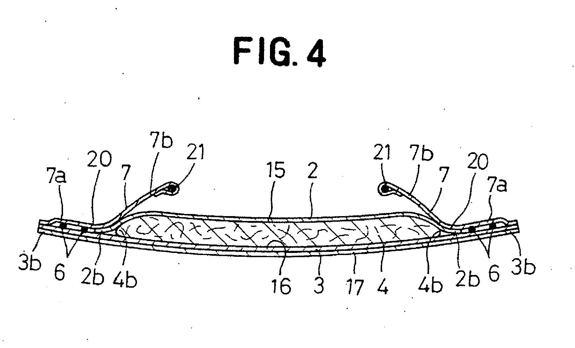

Fig. 1 is a partially cutaway perspective view showing an open-type diaper 1A of the invention, Fig. 2 is a sectional view taken along the line II-II in Fig. 1, Fig. 3 is a sectional view taken along the line III-III in Fig. 1 and Fig. 4 is a sectional view taken along the line IV-IV in Fig. 1. In Fig. 1, a transverse direction is indicated by an arrow L and a longitudinal direction is indicated by an arrow M. As used herein the term "inner surfaces" of top- and backsheets 2, 3 and leak-barrier sheets 7 refers to surfaces thereof facing a core 4 and as used herein the term "outer surfaces" of these sheets 2, 3, 7 refers to surfaces thereof facing away from the core 4. As used herein the term "skin contacting side" refers to the side facing the wearer's skin and expression used herein "skin non-contacting side" refers to the side facing away from the wearer's skin.

The diaper 1A basically comprises the liquid-pervious topsheet 2 lying on the skin contactable side, the liquid-impervious backsheet 3 lying on the skin non-contactable side and the liquid-absorbent core 4 interposed between the top- and backsheets 2, 3. In addition to these constituting members, the diaper 1A includes tape-like waist-surrounding elastic members 5, a plurality of strand-like leg-surrounding elastic members 6 and a pair of the leak-barrier sheets 7.

The diaper 1A is composed, in the longitudinal direction, a front waist region 8, a rear waist region 10 and a crotch region 9 extending between these waist regions 8, 10, and the diaper 1A is contoured by longitudinally opposite end portions 11 extending in the transverse direction outside longitudinally opposite ends 4a of the core 4 and transversely opposite side edge portions 12 extending in the longitudinal direction outside transversely opposite ends 4b of the core 4. The front waist region 8 is provided with a pair of first wings 13 extending outward from respective side edge portions 12 of the front waist region 8 in the transverse direction. The rear waist region 10 is also provided with a pair of second wings 14 extending outward from the respective side edge portions 12 of the rear waist region 10 in the transverse direction. Thus, the diaper 1A has a generally hourglass-like planar shape.

The topsheet 2 is formed from a breathable hydrophilic fibrous nonwoven fabric 15. The backsheet 3 is formed from a breathable liquid-impervious plastic film 16 lying on the lower surface of the core 4 and a breathable hydrophobic fibrous nonwoven fabric 17 placed on the lower surface of the film 16. Respective surfaces of the film 16 and the nonwoven fabric 17 opposed to each other are intermittently bonded by means of adhesive (not shown). The core 4 extends between the front and rear waist regions 8, 10 and is permanently bonded to at least one of inner surfaces of the topsheet 2 and the film 16 of the backsheet 3.

The core 4 comprises a mixture of particulate or fibrous super-absorbent polymer and fluff pulp or a mixture of particulate or fibrous super-absorbent polymer, fluff pulp and thermoplastic synthetic resin fiber, in any case, compressed to a desired thickness. Preferably, the core 4 is entirely wrapped with a liquid-pervious sheet such as a tissue paper or hydrophilic fibrous nonwoven fabric in order to avoid a possibility that the core 4 might get out of its initial shape and/or the particulate polymer might fall off from the core 4. The polymer may be selected from the group consisting of starch-based polymer, cellulose-based polymer and synthetic polymer.

The longitudinally opposite end portions 11 are formed from longitudinally opposite end portions 2a of the topsheet 2 and longitudinally opposite end portions 3a of the backsheet 3 extending outward beyond longitudinally opposite ends 4a of the core 4. The top- and backsheets 2, 3 are overlapped together along the end portions 2a, 3a thereof and inner surfaces of these sheets 2, 3 are permanently bonded together to form the respective end portions 11. The waist surrounding elastic members 5 are contractibly attached to the respective end portions 11 so as to extend in the transverse direction. More specifically, the waist surrounding elastic members 5 are interposed between the respective end portions 2a of the topsheet 2 and the respective end portions 3a of the backsheet 3 and are permanently bonded to the inner surfaces of these sheets 2, 3 while these elastic members 5 are stretched at a given ratio.

The transversely opposite side edge portions 12 are formed from transversely opposite side edge portions 2b of the topsheet 2 and transversely opposite side edge portions 3b of the backsheet 3 extending outward in the transverse direction beyond transversely opposite side edges 4b of the core 4. The side edge portions 2b of the topsheet 2 extend outward in the transverse direction slightly beyond the transversely opposite side edges 4b of the core 4 and the side edge portions 3b of the backsheet 3 as well as the side edge portions 7a of the leak-barrier sheets 7 extend outward in the transverse direction beyond the side edge portions 2b of the topsheet 2. Along the side edge portions 12, the respective side edge portions 2b, 3b, 7a are overlapped together and have inner and outer surfaces thereof bonded together. The leg surrounding elastic members 6 are contractibly attached to the respective side edge portions 12 of the crotch region 9 so as to extend in the longitudinal direction. More specifically, the leg surrounding elastic members 6 are interposed between the respective side edge portions 3b of the backsheet 3 and the respective side edge portions 7a of the leak-barrier sheets 7 and are permanently bonded to the inner surfaces of these sheets 3, 7 while these elastic members 6 are stretched at a given ratio.

The first wings 13 are defined by the side edge portions 3b of the backsheet 3 and the fixed side portions 7a of the respective leak-barrier sheets 7 extending outward from the side edge portions 12 of the front waist region 8 in the transverse direction. These sheets 3, 7 are overlapped together and have respective inner surfaces bonded together along these side edge portions 3b, 7a of the sheets 3, 7.

The second wings 14 are formed from an elastically stretchable composite sheet 18, respectively, which are elastically stretchable in the transverse direction. Each of the second wings 14 has its transversely inner end portion 14a interposed between the side edge portion 3b of the backsheet 3 and the fixed side edge portion 7a of the leak-barrier sheet 7 and permanently bonded to respective inner surfaces of these sheets 3, 7 by means of adhesive (not shown). The transversely outer end portions 14b of the respective second wings 14 are provided with tape fasteners 19 extending in the transverse direction.

The leak-barrier sheets 7 are formed from a breathable hydrophobic fibrous nonwoven fabric 20 and extend in the longitudinal direction between the front and rear waist regions 8, 10. Each of the leak-barrier sheets 7 has the fixed side portion 7a lying outside the associated side edge 4b of the core 4 and extending in the longitudinal direction, a movable portion 7b normally biased to rise up above the topsheet 2 and extending in the longitudinal direction and longitudinally opposite fixed end portions 7c lying on the longitudinally opposite end portions 11, respectively, and collapsed inward in the transverse direction of the diaper 1A. An elastic member 21 extending in the longitudinal direction is contractibly attached to the movable portion 7b in the vicinity of its distal end wherein the elastic member 21 is wrapped with a part of the movable portion 7b and permanently bonded to the movable portion 7b by means of adhesive (not shown). The fixed end portions 7c are permanently bonded to the outer surface of the respective end portions 2a of the topsheet 2. In the leak-barrier sheets 7, the elastic members 21 contract as the diaper 1A is curved in the longitudinal direction with the topsheet 2 inside and a contractile force of these elastic members 21 causes the movable portions 7b to rise above the topsheet 2 so as to form barriers against bodily discharges.

Each of the tape fasteners 19 has a fixed side portion 19a permanently bonded to the outer end portion 14b of the associated second wing 14 by means of adhesive (not shown) and a free portion 19b extending outward from the fixed side portion 19a in the transverse direction. Stock materials for the tape fastener 19 may be selected from the group consisting of a fibrous nonwoven fabric made of polyolefin-based thermoplastic synthetic resin fiber and a film made of polyolefin-based thermoplastic resin. The free portion 19b is provided with a plurality of hooks 22 extending in a thickness direction of the diaper 1A. These hooks 22 are made of polyolefin-based thermoplastic synthetic resin. Alternatively, the free portion 19b may be coated with pressure-sensitive adhesive, instead of the hooks 22.

The front waist region 8 is provided with a target tape 23 on which the free end portions 19b of the respective tape fasteners 19 are releasably anchored. The target tape 23 has a rectangular shape having its longer sides extending in the transverse direction and is intermittently or continuously bonded to the outer surface of the nonwoven fabric 17 of the backsheet 3 by means of adhesive (not shown). Stock materials for this target tape 23 may be selected from the group consisting of a fibrous nonwoven fabric made of polyolefin-based thermoplastic synthetic resin fiber and a film made of polyolefin-based thermoplastic synthetic resin. The target tape 23 is provided on its outer surface with a plurality of loops 24 each describing a circular arc in the thickness direction of the diaper 1A. These loops 24 are made of polyolefin-based thermoplastic synthetic resin. When it is desired to coat the free portions 19b of the respective tape fasteners 19 with pressure-sensitive adhesive, the synthetic resin film may be used as the target tape 23.

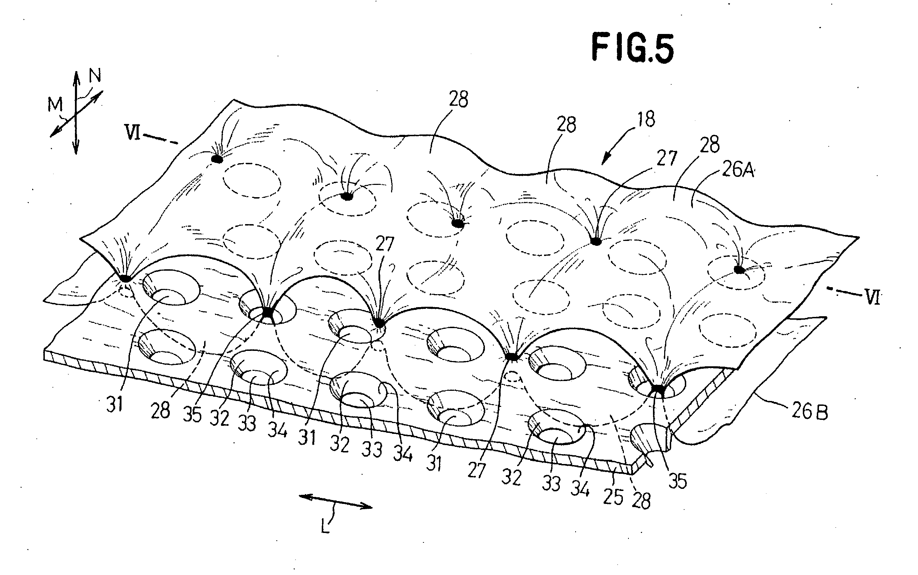

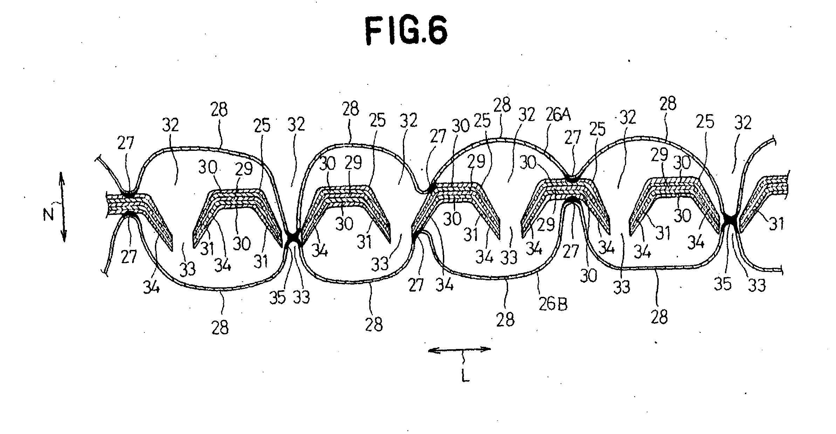

Fig. 5 is a partially cutaway perspective view showing a composite sheet 18 forming the second wings 14 and Fig. 6 is a section view taken along the line VI-VI. In Figs. 5, 6, a transverse direction is indicated by an arrow L, a longitudinal direction is indicated by an arrow M and a thickness direction is indicated by an arrow N. The composite sheet 18 comprises a breathable and stretchable plastic film 25 and a pair of breathable and heat-sealable fibrous nonwoven fabric layers 26A, 26B. The plastic film preferably has a thickness of 0.3 - 1 mm, the nonwoven fabric layers 26A, 26B preferably has a basic weight of 20 - 60 g/m², a thickness of 0.1 - 0.5 mm and a density in a range of 0.04 - 0.6 g/cm³, respectively. The nonwoven fabric layers 26A lies on the skin contactable side and the nonwoven fabric layers 26B lies on the skin non-contactable side. The plastic film 25 and the nonwoven fabric layers 26A, 26B have respective surfaces overlapped and partially bonded together by means of a plurality of heat-sealed spots 27 which are distributed as evenly as possible. These nonwoven fabric layers 26A, 26B are formed with a plurality of gathers 28 which rise and fall along surfaces of the nonwoven fabric layers 26A, 26B. The nonwoven fabric layers 26A, 26B may be hydrophilic or hydrophobic.

The plastic film 25 is formed from an elastically stretchable thermoplastic elastomer film 29 and thermoplastic synthetic resin film layers 30 placed upon both surfaces of the elastomer film 29. The elastomer film 29 and the synthetic resin film layers 30 are overlapped and bonded together along respective surfaces thereof opposed one to another. Each of the thermoplastic synthetic resin film layers 30 comprises a pair of two sub-layers overlapped and bonded together. The plastic film 25 is formed with a plurality of air passages 31 extending from its upper surface toward its skin non-contactable side (i.e., toward the nonwoven fabric layer 26B) in the thickness direction of the diaper 1A. Each of these air passages 31 is defined by a first opening 32 facing the wearer's skin, a second opening 33 facing away from the wearer's skin and having a diameter smaller than that of the first opening 32 and a tubular wall 34 extending between the first and second openings 32, 33 so as to be tapered from the first opening 32 toward the second opening 33.

Inside some of the air passage 31, the nonwoven fabric layers 26A, 26B have respective surfaces facing each other bonded together by means of heat-sealed spots 35. Inside the remaining air passages 31, however, these nonwoven fabric layers 26A, 26B are not heat-sealed together. At the heat-sealed spots 27, 35, these nonwoven fabric layers 26A, 26B are not in a filmy state but maintain a fibrous state. In other words, a fiber density of the nonwoven fabric layers 26A, 26B is higher than a fiber density thereof in the remaining region except the heat-sealed spots 27, 35.

The elastomer film 29 is made of material selected from the group consisting of styrene-based block copolymer, polyurethane-based block copolymer, polyester-based block copolymer, polyamide-based block copolymer and copolymer blend. The styrene-based block copolymer may be selected from a group consisting of styrene-butadiene-styrene (S-B-S) and styrene-ethylene butadiene-styrene (S-EB-S). The copolymer blend may be selected from a group consisting of styrene-ethylene butadiene-styrene/polypropylene (S-EB-S/PP) and polypropylene/ethylene-propylene (PP/-P).

The synthetic resin film 30 is formed from polyolefin-based thermoplastic synthetic resin. The fibrous nonwoven fabric is formed from polyolefin-based thermoplastic synthetic resin fiber. The polyolefin may be selected from the group consisting of polyamide, polyester, polyvinyl chloride, polyethylene, polypropylene and polystyrene.

The plastic film 25 may be obtained, for example, by using a process including the steps as follow. The elastomer film 29 and the synthetic resin film 30 are respectively extruded from the associated extruders and placed upon each other before cured so that respective surfaces of these film layers 29, 30 opposed to each other may be heat-sealed so as to form the plastic film 25. In this step, the plastic film 25 is not formed with the air passages 31.

In the softened state, the plastic film 25 runs to a rotary suction drum and one surface of the plastic film 25 is held in contact with a peripheral surface of the drum as the plastic film 25 travels on the peripheral surface. The suction drum is provided on its peripheral surface with a plurality of openings distributed as evenly as possible and tubular walls extending from peripheral edges of the respective openings toward an axis of the drum. The suction drum is provided on its peripheral surface with a suction station at which the air is sucked by suction means toward the axis of the drum through the respective openings. Regions of the plastic film 25 just facing the respective openings of the drum are sucked and stretched from the openings toward the axis of the drum as the plastic film 25 traveling on the peripheral surface of the drum reaches the suction station. In consequence, lower ends of the respective regions of the plastic film 25 sucked and stretched in this manner and exposed to the internal space of the drum are disrupted to form a plurality of air passages 31 extending toward the axis of the drum. Then the plastic film 25 is cooled and cured and taken up on a roll.

The stretchable composite sheet 18 may be obtained, for example, by using a process including the steps as follows. The nonwoven fabric layers 26A, 26B substantially in non-stretched state are placed upon both surfaces of the plastic film 25 being stretched in the transverse direction. Then these film 25 and the nonwoven fabric layers 26A, 26B are partially heated to bond them together and, at the same time, to bond the nonwoven fabric layers 26A, 26B to each other inside the air passages 31. Upon relaxation of the plastic film 25, the film 25 contracts in the transverse direction and the nonwoven fabric layers 26A, 26B also contract in the transverse direction to form a plurality of gathers 28. Bonding of the film 25 to the nonwoven fabric layers 26A, 26B may be achieved using thermal embossing technique or ultrasonic sealing technique. It should be noted here that the nonwoven fabric layers 26A, 26B are bonded to each other inside some of the air passages 31 but not bonded to each other inside the remaining air passages 31.

An example of the thermal embossing technique uses a thermal embossing roll provided on its peripheral surface with a plurality of projections and a flat roll opposed to the embossing roll so that the film 25 and the nonwoven fabric layers 26A, 26B placed one upon another may be guided into a nip of these rolls. In the course of passing through the nip, the film 25 is stretched in a machine direction at a given ratio. The nonwoven fabric layers 26A, 26B are under a tension in the machine direction but not stretched. Passing through the nip of these paired rolls, the film 25 and the nonwoven fabric layers 26A, 26B are wedged between the embossing projections and the peripheral surface of the flat roll and heated by the embossing projections. In this way, these film 25 and nonwoven fabric layers 26A, 26B are partially heat sealed together.

The ultrasonic sealing technique generally uses a horn and an anvil opposed to each other so that the film 25 and the nonwoven fabric layers 26A, 26B placed one upon another may be guided through a gap between these horn and anvil. In the course of passing through the gap, the film 25 is stretched in the machine direction at a given ratio. The nonwoven fabric layers 26A, 26B are under a tension in the machine direction but not elastically stretched. Frictional heat generated between the horn and the anvil both oscillating under the effect of ultrasonic waves causes the film 25 and the nonwoven fabric layers 26A, 26B to be partially heat-sealed together.

The film 25 and the nonwoven fabric layers 26A, 26B are preferably formed from the same type of polyolefin-based material. For example, when polyethylene-based synthetic resin is used to form the film 25, it is preferred to use polyethylene-based synthetic resin fiber as the component fiber of the nonwoven fabric layers 26A, 26B. The film 25 and the nonwoven fabric layers 26A, 26B formed from the same type of polyolefin-based material present respective melting points which are substantially similar one to another and can be easily bonded one to another. When the film 25 and the nonwoven fabric layers 26A, 26B are formed from different types of polyolefin-based material, respectively, it is preferred to use the material having a melting point substantially the same as or lower than that of the film 25 for the component fiber of the nonwoven fabric layers 26A, 26B. This is because, if the film 25 has a melting point higher than the melting point of the nonwoven fabric layers 26A, 26B, the nonwoven fabric layers 26A, 26B will inevitably become filmy in the course of heat-sealing them together with the film 25 and it will be difficult for the nonwoven fabric layers 26A, 26B to maintain their fibrous state at the heat-sealed spots 27, 35.

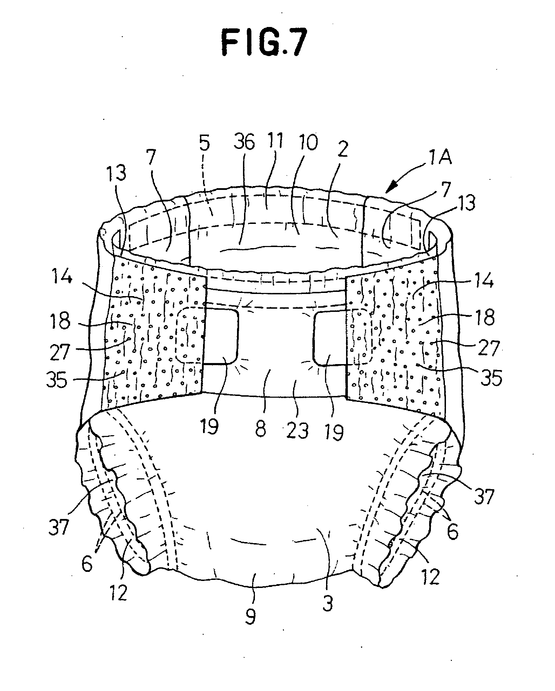

Fig. 7 is a perspective view showing the diaper 1A as put on the wearer's body. An example of the sequence in accordance with which a parent or a care personnel puts the diaper 1A on the wearer's body will be described. After the hip of the wearer lying face up has been laid on the developed diaper 1A, the front waist region 8 is held with the hand and folded back along the crotch region 9 onto the wearer's belly. Then, with the tape fasteners 19 gripped by the fingers, the second wings 14 are folded back so that these wings 14 may be positioned above the wearer's belly. The second wings 14 are placed on respective outer sides of the end portion 11 and the first wings 13 of the front waist region 8. Adjusting a tightness of the end portions 11 as well as of the wings 14 around the wearer's waist, the free portions 19b of the respective tape fasteners 19 are anchored on the outer surface of the target tape by means of the hooks 22 and thereby the front and rear waist regions 8, 10 are connected to each other.

To anchor the tape fasteners 19 on the target tape 23, the free portions 19b of the respective tape fasteners 19 may be pressed against the outer surface of the target tape 23 and thereby the hooks 22 may be engaged with the loops 24. Upon connection of the front and rear waist regions 8, 10 to each other, the diaper 1A is formed with a waist-hole 36 and a pair of leg-holes 37. Excretion discharged on the diaper 1A put on the wearer's body is absorbed by the core 4 through the topsheet 2 and retained therein.

Of the composite sheet 18 forming each of the second wings 14, the fibrous nonwoven fabric layer 26A contacts the wearer's skin during use of the diaper 1A, so it is not apprehended that the wings 14 might uncomfortably stick to the wearer's skin in spite of stretchable elasticity of the wings 14. Comfortable feeling to wear the diaper 1A is further enhanced by forming the nonwoven fabric layers 26A, 26B using polyolefin-based synthetic resin fiber which is free from tackiness as well as sliminess peculiar to rubber-based material. Tightness of the diaper 1A around the wearer's waist can be appropriately adjusted by utilizing the elasticity of the second wings 14 so as to prevent the diaper 1A from slipping down from its proper position.

The stretchable plastic film 25 constituting the composite sheet 18 is formed with a plurality of air passages 31 so that water vapor generated within the diaper 1A due to sweat evaporation can be let out from the diaper 1A through these air passages 31 and thereby stuffiness possibly occurring within the diaper 1A can be prevented. More specifically, each of these air passages 31 extending through the plastic film 25 in its thickness direction is tapered from the first opening 32 toward the second opening 33 and a surface tension acting on the air passage 31 is higher in the vicinity of the second opening 33 than in the vicinity of the first opening 32. Consequently, sweat having flowed into the first opening 32 can be easily transferred toward the second opening 33, i.e., toward the skin non-contactable side of the second wing 14 under the surface tension. In this way, wetness on this side of the wing 14 can be rapidly eliminated.

A total thickness of the film 25 and the nonwoven fabric layers 26A, 26B in the heat-sealed spots 27 is smaller than that of the film 25 and the nonwoven fabric layer 26A except the heat-sealed spots 27, so the nonwoven fabric layer 25A except the head-sealed spots 27 comes in contact with the wearer's skin as the diaper 1A is put on. The nonwoven fabric layers 26A, 26B have fiber densities higher in the heat-sealed spots 27, 35 than in the respective remaining regions and therefore a capillary phenomenon occurs more significantly in the heat-sealed spots 27, 35 than in the remaining regions. Such significant capillary phenomenon allows any amount of sweat having permeated the remaining region of the nonwoven fabric layer 26A to be rapidly transferred to the heat-sealed spots 27, 35 and thereby allows the remaining region to be rapidly dried. In this way, uncomfortable tackiness and/or sliminess due to sweat which would otherwise stay on the remaining region of the nonwoven fabric layer 26A contacting with the wearer's skin can be effectively eliminated. Inside some of the air passages 31, the nonwoven fabric layers 26A, 26B are bonded together at the heat-sealed spots 35, so water vapor generated as the amount of sweat having moved to the heat-sealed spots 35 is evaporated can be let out from the diaper through these air passages 31 and the amount of sweat having permeated the nonwoven fabric layer 26A can be rapidly dried.

The number of the air passages 31 formed through the plastic film 25 is in a range of 10 - 80/cm² of the plastic film 25 and a total area ratio of the first openings 32 of these air passages 31 is in a range of 10 - 20% of the plastic film 25. If the number of the air passages 31 is less than 10 and the total area ratio of the first openings 32 of these air passages 31 is less than 10% of an area of the plastic film 25, the number of the air passages 31 per square centimetre of the plastic film 25 as well as the opening area of the first openings 32 will be insufficient to let the water vapor out from the diaper 1A and to transfer the sweat toward the skin non-contacting side of the second wings 14. As a result, it would be impossible to prevent a stuffiness from being generated within the diaper 1A. If the number of the air passages 31 exceeds 80/cm² and the total area ratio of the first openings 32 of these air passages 31 exceeds 20% of the area of the plastic film 25, on the contrary, the film 25 will be formed with a number of the first openings 32 excessively close one to another and the film 25 will be broken between each pair of the adjacent first openings 31. Thereupon the second wings 14 will lose the desired elasticity and no more contract again. The nonwoven fabric layers 26A, 26B are bonded to each other inside the air passages 31 formed through the film 25 by a range of 1.5 - 10% with respect to the total number of the air passages 31. If this percentage is less than 1.5%, it will be impossible to let the water vapor generated due to evaporation of the sweat having been transferred to the heat-sealed spots 35 out from the diaper 1A and therefore to dry the sweat having permeated the nonwoven fabric layer 26A.

Each of the second wings 14 formed from the elastic composite sheet 18 presents a transverse stretch stress of 1.5 - 7.0 N as the wing 14 is stretched in the transverse direction to a length dimension in the transverse direction corresponding to 120% of its non-stretched length dimension set as 100% and presents a transverse stretch stress of 3.0 - 10.0 N as the wing 14 is stretched to a length dimension in the transverse direction corresponding to 150% of its non-stretched length dimension. If the stretch stress of the second wing 14 exceeds the above-mentioned higher limits, the wings 14 will needlessly clinch the wearer's waist as the diaper 1A is put on and the wearer will experience a feeling of oppression partially around his or her waist. If the stretch stress of the second wing 14 is less than the above-mentioned lower limits, on the contrary, a contractile force of the wing 14 will be substantially ineffective and it will be impossible for the wings 14 to clinch the wearer's waist appropriately. The stretch stress was measured using a method as follows:
(1) The transversely opposite side edge portions 12 of the rear waist region 10 are cut away from the diaper 1A to prepare samples for measurement of stretch stress. Each sample comprises a part of the side edge 12 and the complete second wing 14 (including the tape fastener 19). INSTRON 5543 or AUTOGRAPH manufactured by INSTRON JAPAN CO. is used to measure the stretch stress.
(2) A fixture is attached to the side edge portion 12 of the sample except the second wing 14 so that the fixture may extend along full length of the side edge portion 12. Of the sample, the tape fastener 19 is held by an upper chuck and the fixture is held by a lower chuck. A length dimension of the sample measured between the upper and lower chucks is 70 mm.
(3) A length dimension of the non-stretched sample extending between the upper and lower chucks without slacks is set as 100%. The sample is stretched in the vertical direction at a rate of 100 min/min so that the upper and lower chucks may get away from each other. The sample is stretched to a length dimension corresponding to 120% of its non-stretched length and a stretch stress of the sample at this moment is measured and then the sample is stretched to a length dimension corresponding to 150% of its non-stretched length and a stretch stress of the sample at this moment is measured.

### EMBODIMENT 2

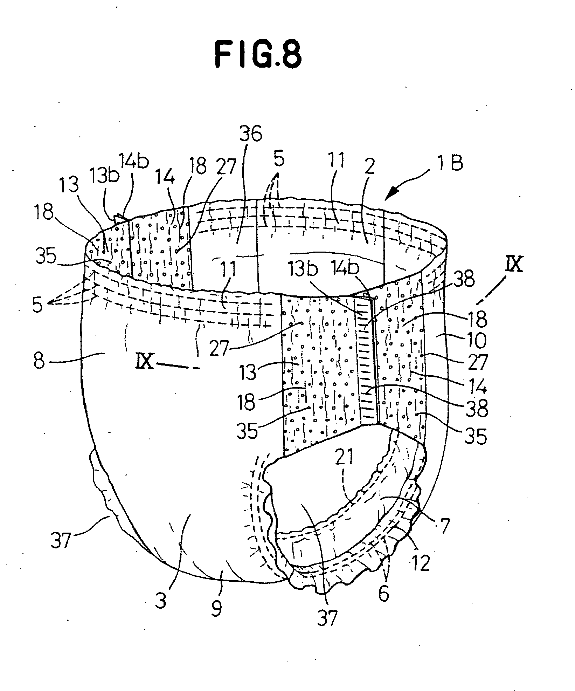

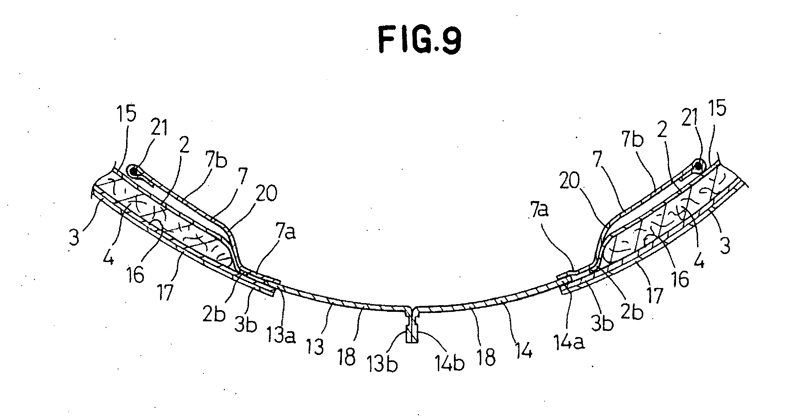

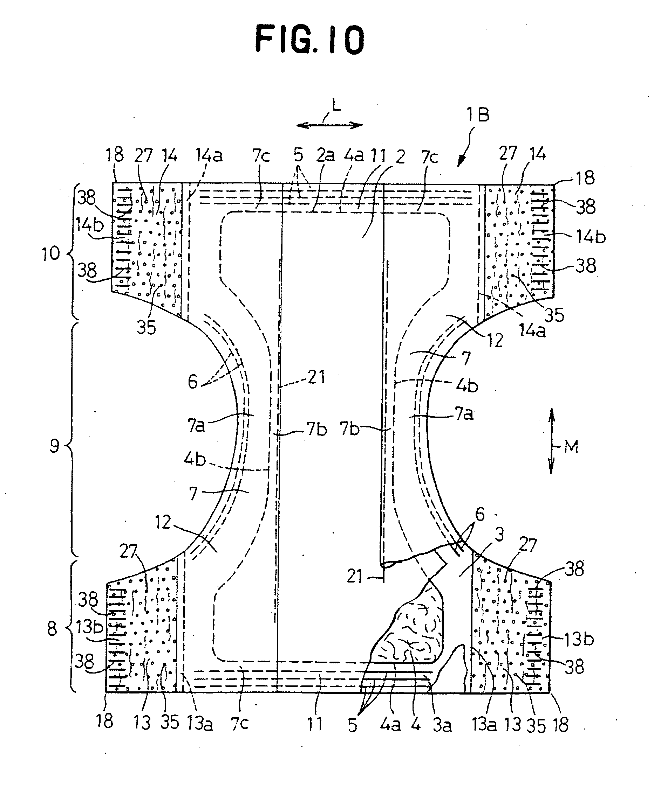

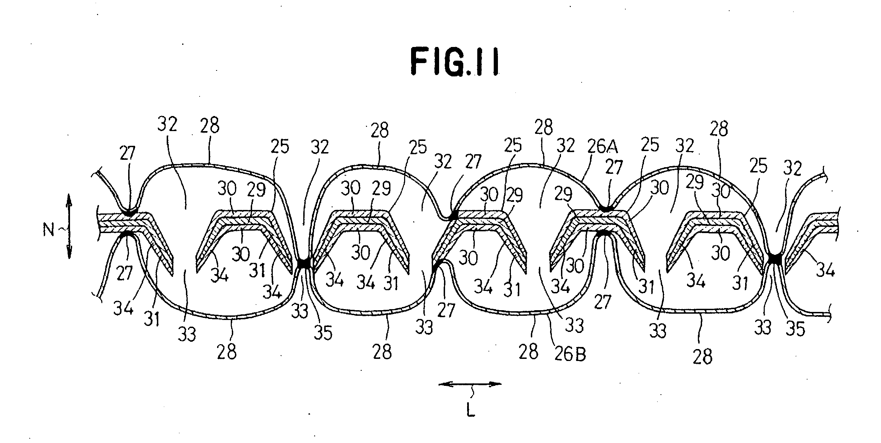

Fig. 8 is a perspective view showing a pull-on diaper 1B as another embodiment of the invention, Fig. 9 is a sectional view taken along the line IX-IX in Fig. 8, Fig. 10 is a developed view showing the diaper 1B of Fig. 8 with the front and rear waist regions 8, 10 having been disconnected from each other as partially broken away and Fig. 11 is an end view showing the composite sheet forming the first and second wings 13, 14. In Figs. 8 and 10, the transverse direction is indicated by an arrow L and the longitudinal direction is indicated by an arrow M. In Fig. 11, the transverse direction is indicated by an arrow L and the thickness direction is indicated by an arrow N.

The diaper 1B basically comprises a liquid-pervious topsheet 2, a liquid-impervious backsheet 3 and a liquid-absorbent core 4 interposed between the top- and backsheets 2, 3. The diaper 1B further comprises a plurality of strand-like waist surrounding elastic members 5 and leg surrounding elastic members 6 and a pair of leak-barrier sheets 7. As will be understood from Fig. 10, the diaper 1B has a generally hourglass-like developed planar shape.

The diaper 1B is composed of front and rear waist regions 8, 10 opposed to each other and a crotch region 9 extending between these waist regions 8, 10 wherein the diaper 1B is contoured by longitudinally opposite end portions 11 extending in the transverse direction outside longitudinally opposite ends 4a of the core 4 and transversely opposite side edge portions 12 extending in the longitudinal direction outside transversely opposite side edges 4b of the core 4. The front waist region 8 is provided with a pair of first wings 13 extending outward from respective side edge portions 12 of the front waist region 8 in the transverse direction. The rear waist region 10 is provided with a pair of second wings 14 extending outward from the respective side edge portions 12 of the rear waist region 10 in the transverse direction. In the diaper 1B, laterally outer end portions of the first and second wings 13, 14 are put flat and permanently bonded together at a plurality of heat-sealing spots 38 arranged intermittently in the longitudinal direction. Thereupon the diaper 1B is formed with a waist-hole 36 and a pair of leg-holes 37.

The topsheet 2 is formed from the same nonwoven fabric 15 as that of the diaper 1A and the backsheet 3 is formed from the plastic film 16 and the nonwoven fabric 17 which are the same as those of the diaper 1A. The core 4 is the same as that of the diaper 1A and permanently bonded to the inner surface of at least one of the top- and backsheets 2, 3.

The longitudinally opposite end portions 11 are formed from longitudinally opposite end portions 2a of the topsheet 2 and longitudinally opposite end portions 3a of the backsheet 3 extending outward beyond longitudinally opposite ends 4a of the core 4. The top- and backsheets 2, 3 are overlapped together along the end portions 2a, 3a thereof and inner surfaces of these sheets 2, 3 are permanently bonded together to form the respective end portions 11. The waist surrounding elastic members 5 are attached to the inner surfaces of the top- and backsheets 2, 3 so as to be interposed between the end portions 2a of the topsheet 2 and the end portions 3a of the backsheet 3 while these elastic members 5 are stretched at a given ratio in the transverse direction.

The transversely opposite side edge portions 12 are formed from transversely opposite side edge portions 2b of the topsheet 2 and transversely opposite side edge portions 3b of the backsheet 3 extending outward in the transverse direction beyond transversely opposite side edges 4b of the core 4. The side edge portions 2b of the topsheet 2 extend outward in the transverse direction slightly beyond the transversely opposite side edges 4b of the core 4 and the side edge portions 3b of the backsheet 3 as well as the side edge portions 7a of the leak-barrier sheets 7 extend outward in the transverse direction beyond the side edge portions 2b of the topsheet 2. Along the side edge portions 12, the respective side edge portions 2b, 3b, 7a are overlapped and permanently bonded together. The leg surrounding elastic members 6 are interposed between the side edge portions 3b of the backsheet 3 and the side edge portions 7a of the respective leak-barrier sheets 7 and permanently bonded to the respective inner surfaces of these sheets 3, 7 while these elastic members 6 are stretched at a given ratio in the longitudinal direction.

The leak-barrier sheets 7 are formed from the same nonwoven fabric 20 as that of the diaper 1A and extend in the longitudinal direction between the front and rear waist regions 8, 10. Each of the leak-barrier sheets 7 has the fixed side portion 7a lying outside the associated side edge 4b of the core 4 and extending in the longitudinal direction, a movable portion 7b normally biased to rise above the topsheet 2 and longitudinally opposite fixed end portions 7c lying on the longitudinally opposite end portions 11, respectively, and collapsed inward in the transverse direction of the diaper 1B. An elastic member 21 extending in the longitudinal direction is contractibly attached to the movable portion 7b in the vicinity of its distal end. The movable portion 7b rises up above the topsheet 2 so as to form barriers against bodily discharges.

The first and second wings 13, 14 have transversely inner end portions 13a, 14a interposed between the side edge portions 3b of the backsheet 3 and the fixed side portions 7a of the leak-barrier sheets 7 and permanently bonded to the respective inner surfaces of these sheets 3, 7 by means of adhesive (not shown). The first and second wings 13, 14 are formed from an elastically stretchable composite sheet 18, respectively, which are elastically stretchable in the transverse direction.

The composite sheet 18 comprises a breathable and stretchable plastic film 25 and a pair of breathable and heat-sealable fibrous nonwoven fabric layers 26A, 26B. The plastic film 25 and the nonwoven fabric layers 26A, 26B have respective surfaces overlapped and partially bonded together by means of a plurality of heat-sealed spots 27 which are distributed as evenly as possible. These nonwoven fabric layers 26A, 26B are formed with a plurality of gathers 28 which rise and fall in the thickness direction of the diaper 1B. The nonwoven fabric layers 26A, 26B may be hydrophilic or hydrophobic.

The plastic film 25 is formed from a elastically stretchable thermoplastic elastomer film 29 and thermoplastic synthetic resin film layers 30 placed upon both surfaces of the elastomer film 29. The elastomer film 29 and the synthetic resin film layers 30 are overlapped and bonded together along respective surfaces thereof opposed one to another. The plastic film 25 is formed with a plurality of air passages 31 extending from its upper surface toward its skin non-contactable side (i.e., toward the nonwoven fabric layer 26B) in the thickness direction of the diaper 1B. Each of these air passages 31 is defined by a first opening 32 facing the wearer's skin, a second opening 33 facing away from the wearer's skin and having a diameter smaller than that of the first opening 32 and a tubular wall 34 extending between the first and second openings 32, 33 so as to be tapered from the first opening 32 toward the second opening 33.

Inside the air passage 31, the nonwoven fabric layers 26A, 26B have respective surfaces facing each other bonded together by means of heat-sealed spots 35. Similarly to the case of the second wings 14 shown in Fig. 1, these nonwoven fabric layers 26A, 26B are bonded to each other in some of the air passages 31. At the heat-sealed spots 27, 35, these nonwoven fabric layers 26A, 26B at the heat-sealed spots 27, 35 maintain a fibrous state. In other words, a fiber density of the nonwoven fabric layers 26A, 26B at these heat-sealed spots 27, 35 is higher than a fiber density thereof in the remaining region.

The elastomer film 29 and the synthetic resin film 30 are formed from the same materials as those of the diaper 1A, respectively. The process for making the stretchable composite sheet 18 as well as the process for making the plastic film 25 are the same as those having been described in reference with the diaper 1A. To shape the diaper 1B illustrated by Fig. 8 from its state illustrated by Fig. 10 in the developed plan view, the diaper 1B may be folded along the crotch region 9 with the topsheet inside so that the front and rear waist regions 8, 10 are opposed to each other and the outer end portions 13b, 14b of the first and second wings 13, 14 may be permanently bonded together by means of the heat-sealing lines 38.

Of the composite sheet 18 forming each of the second wings 14, the fibrous nonwoven fabric layer 26A contacts the wearer's skin during use of the diaper 1B, so it is not apprehended that the wings 14 might uncomfortably stick to the wearer's skin in spite of stretchable elasticity of the wings 14. Comfortable feeling to wear the diaper 1B is further enhanced by forming the nonwoven fabric layers 26A, 26B using polyolefin-based synthetic resin fiber which is free from tackiness as well as sliminess peculiar to rubber-based material. Tightness of the diaper 1B around the wearer's waist can be appropriately adjusted by utilizing the elasticity of the second wings 14 so as to prevent the diaper 1B from slipping down from its proper position.

The stretchable plastic film 25 constituting the composite sheet 18 is formed with a plurality of air passages 31 so that water vapor generated within the diaper 1B due to sweat evaporation can be let out from the diaper 1B through these air passages 31 and thereby stuffiness possibly occurring within the diaper 1B can be prevented. More specifically, each of these air passages 31 extending through the plastic film 25 in its thickness direction is tapered from the first opening 32 toward the second opening 33 and a surface tension acting on the air passage 31 is higher in the vicinity of the first opening 32 than in the vicinity of the second opening 33. Consequently, sweat having flowed into the first opening 32 can be easily transferred toward the second opening 33, i. e. , toward the skin non-contacting side of the second wing 14 under the surface tension. In this way, wetness on this side of the wing 14 can be rapidly eliminated.

The nonwoven fabric layers 26A, 26B have fiber densities higher in the heat-sealed spots 27, 35 than in the respective remaining regions and therefore a capillary phenomenon occurs more significantly in the heat-sealed spots 27, 35 than in the remaining regions. Such significant capillary phenomenon allows any amount of sweat having permeated the remaining region of the nonwoven fabric layer 26A to be rapidly transferred and thereby allows the remaining region to be rapidly dried. In this way, uncomfortable tackiness and/or sliminess due to sweat which would otherwise stay on the remaining region of the nonwoven fabric layer 26A contacting with the wearer's skin can be effectively eliminated. Inside some of the air passages 31, the nonwoven fabric layers 26A, 26B are bonded together at the heat-sealed spots 35, so water vapor generated as the amount of sweat having moved to the heat-sealed spots 35 is evaporated can be let out from the diaper 1B through these air passages 31 and the amount of sweat having permeated the nonwoven fabric layer 26A can be rapidly dried.

The number of the air passages 31 formed through the plastic film 25 is in a range of 10 - 80/cm² in the plastic film 25 and a total area ratio of the first openings 32 of these air passages 31 is in a range of 10 - 20% of the plastic film 25. If the number of the air passages 31 is less than 10% and the total area ratio of the first openings 32 of these air passages 31 is less than 10% of an area of the plastic film 25, the number of the air passages 31 per square centimetre of the plastic film 25 as well as the opening area of the first openings 32 will be insufficient to let the water vapor out from the diaper 1B and to transfer the sweat toward the skin non-contacting side of the second wings 14. As a result, it would be impossible to prevent a stuffiness from being generated within the diaper 1B. If the number of the air passages 31 exceeds 80/cm² and the total area ratio of the first openings 32 of these air passages 31 exceeds 20% of the area of the plastic film 25, on the contrary, the film 25 will be formed with a number of the first openings 32 excessively close one to another and the film 25 will be broken between each pair of the adjacent first openings 32. Thereupon the second wings 14 will lose the desired elasticity and no more contract again. In the diaper 1B, the nonwomen fabric layers 26A, 26B are bonded to each other inside the passages 31 formed through the film 25 by a range of 1.5 - 10% with respect to all the air passages 31. If this percentage is less than 1.5%, it will be impossible to let the water vapor generated due to evaporation of the sweat having been transferred to the heat-sealed spots 35 out from the diaper 1B and therefore to dry the sweat having permeated the nonwoven fabric layer 26A.

The first and second wings 13, 14 formed from a stretchable composite sheet 18 present transverse stretch stresses of 1.5 - 7.0 N as the wings 13, 14 are stretched in the transverse direction to length dimensions in the transverse direction corresponding to 120% of non-stretched length dimensions of the respective wings 13, 14 both set as 100% and present transverse stretch stresses of 3.0 - 10.0 N as the wings 13, 14 are stretched to length dimensions in the transverse direction corresponding to 150% of the non-stretched length dimensions of the respective wings 13, 14. If the stretch stresses of the first and second wings 13, 14 exceed the above-mentioned higher limits, the wings 13, 14 will needlessly clinch the wearer's waist as the diaper 1B is put on and the wearer will experience a feeling of oppression partially around his or her waist. If the stretch stresses of the first and second wings 13, 14 are less than the above-mentioned lower limits, on the contrary, contractile force of the wings 13, 14 will be substantially ineffective and it will be impossible for these wings 13, 14 to clinch the wearer's waist appropriately. The stretch stresses of the first and second wings 13, 14 are measured using the same method as has been described in reference with the diaper 1A.

Stock material for the topsheet 2 may be selected from the group consisting of a hydrophobic fibrous nonwoven fabric having a plurality of apertures and a plastic film having a plurality of fine perforations. Stock material for the backsheet 3 may be selected from the group consisting of a breathable hydrophobic fibrous nonwoven fabric, a breathable liquid-impervious plastic film and a composite nonwoven fabric comprising two or more breathable hydrophobic fibrous nonwoven fabric layers laminated one upon another. Stock materials for the leak-barrier sheets 7 may be selected from the group consisting of a composite nonwoven fabric comprising two or more breathable hydrophobic fibrous nonwoven fabric layers laminated one upon another and a composite sheet comprising breathable hydrophobic fibrous nonwoven fabric and a breathable liquid-impervious plastic film laminated upon each other.

As stock material for the backsheet 3 and the leak-barrier sheets 7, it is also possible to use a composite nonwoven fabric (SM nonwoven fabric or SMS nonwoven fabric) consisting of a melt blown fibrous nonwoven fabric having a high water-resistance and a spun bond fibrous nonwoven fabric having a high strength as well as a high flexibility laminated on at least one side of the melt blown fibrous nonwoven fabric.

Fibrous nonwoven fabrics used to form the top- and backsheets 2, 3, the leak-barreir sheets 7, the first and second wings 13, 14, the tape fasteners 19 and the target tape 23 may be selected from those obtained by spun lace-, needle punch-, melt blown-, thermal bond-, spun bond- and chemical bond-processes.

The hydrophilic fibrous nonwoven fabric may be made of any one of synthetic fiber, semi-synthetic fiber and regenerated fiber each modified to become hydrophilic or conjugate fiber thereof. The hydrophobic fibrous nonwoven fabric may be formed from synthetic fiber and may contain therein semi-synthetic fiber or regenerated fiber both treated to become water repellent. While not specified, the synthetic fiber may be selected from the group consisting of polyester-, polyacrylonitrile-, polyvinyl chloride-, polyethylene-, polypropylene- and polystyrene-based fibers. The suitably useful synthetic fibers further include core-sheath type conjugate fiber, side-by-side type conjugate fiber, macaroni fiber, microporous fiber and bonded-type conjugate fiber.

It is preferred to use hot melt adhesive as the adhesive. In addition to hot melt adhesive, it is also possible to use any one of acrylic adhesive and elastomeric adhesive. The adhesive may be applied on the top- and backsheets 2, 3 and the leak-barrier sheets 7 in a suitable pattern selected from the group consisting of a spiral pattern, zigzag pattern, dotted pattern and striped pattern. Application of the adhesive on these sheets 2, 3, 7 in such pattern generates adhesive-coated regions and adhesive-free regions, resulting in that these sheets 2, 3, 7 are permanently bonded one to another in intermittent fashion, the sheets 2, 3, 7 are bonded to one to another in an intermittent fashion and the core 4 is permanently bonded to the sheets 2, 3.

In the diaper 1A, not only the second wings 14 but also the first wings 13 may be formed from the stretchable composite sheet 18 and, in the diaper 1B, it is possible to use the stretchable composite sheet 18 only for the second wings 14.

## Claims

1. A disposable diaper(1A,1B) comprising:
a front waist region (8);
a rear waist region (10);
a crotch region (9) extending between these two waist regions (8,10);
a pair of first wings (13) extending outward from transversely opposite side edge portions (13a) of said front waist region (8) in a transverse direction;
a pair of second wings (14) extending outward from transversely opposite side edge portions (14a) of said rear waist region (10) in the transverse direction;
of said first and second wings (13, 14), at least said second wings (14) are formed from an elastically stretchable composite sheet (18) which comprises, in turn, a breathable stretchy plastic film (25) formed with a plurality of air passages (31) extending through said plastic film (25) each having a first opening (32) facing the wearer's skin and a second opening (33) facing away from the wearer's skin;
a pair of breathable, heat-sealable fibrous nonwoven fabric layers (26) partially bonded to both surfaces of said plastic film at heat-sealed spots (27,35), said diaper (1A, 1B) being **characterized in that**:
said fibrous nonwoven fabric layers (26) are formed with a plurality of gathers (28) rising and falling along surfaces of said composite sheet (18);
the number of said air passages (31) formed through said plastic film (25) is in a range of 10 - 80/cm² of said plastic film (25) and a total area ratio of said first openings (33) is in a range of 10 - 20% of the plastic film (25) ;
inside said air passage (31), said fibrous nonwoven fabric layers (26) are bonded together at said heat-sealed spots (35) and the number of said air passages (31) inside which said fibrous nonwoven fabric layers (26) are bonded to each other is in a range of 1.5 - 10%; and
each of said wings(14) formed from said elastically stretchable composite sheet (18) presents a transverse stretch stress of 1.5 - 7.0 N as said wing (14) is stretched in the transverse direction to a length dimension in the transverse direction corresponding to 120% of its non-stretched transverse length dimension set as 100% and presents a transverse stretch stress of 3.0 - 10.0 N as said wing (14) is stretched to a length dimension in the transverse direction corresponding to 150% of said non-stretched length dimension.

2. The diaper (1A,1B) according to Claim 1, wherein said plastic film (29) is formed from elastically stretchable thermoplastic elastomer film (29) and a pair of polyolefin-based thermoplastic synthetic resin film layers (39) bonded to both surfaces of said elastomer film (39) and wherein said fibrous nonwoven fabric (26) is formed from polyolefin-based thermoplastic synthetic resin fiber.

3. The diaper (1A,1B) according to Claim 1 or wherein said second opening (34) has an area smaller than that of said first opening (33) so that said air passage (32) is tapered from said first opening (33) toward said second opening (34).

4. The diaper (1A, 1B) according to any one of Claims 1 through 3,wherein said fibrous nonwoven fabric layers (26) maintain a fibrous state at said heat-sealed spots (27,35) and have a fibrous density higher at said heat-sealed spots (27, 35) than in the remaining region.

5. The diaper (1A, 1B) according to any one of Claims 1 through 4 , wherein said plastic film (25) has a thickness of 0.3 - 1 mm, and said fibrous nonwoven fabric layers (26) each has a basic weight of 20 - 60 g/m², a thickness of 0.1 - 0.5 mm and a density of 0.04 - 0.6 g/cm³.

## Patentansprüche

1. Einwegwindel (1A, 1 B), umfassend:
einen vorderen Hüftbereich (8);
einen hinteren Hüftbereich (10);
einen Schrittbereich (9), der sich zwischen diesen beiden Hüftbereichen (8, 10) erstreckt;
ein Paar erster Flügel (13), die sich von quer gegenüberliegenden Seitenrandteilen (13a) des vorderen Hüftbereichs (8) aus in einer Querrichtung erstrecken;
ein Paar zweiter Flügel (14), die sich von quer gegenüberliegenden Seitenrandteilen (14a) des hinteren Hüftbereichs (10) aus in der Querrichtung erstrecken;
wobei von den ersten und den zweiten Flügeln (13, 14) mindestens die zweiten Flügel (14) aus einer elastisch dehnbaren Verbundstoffbahn (18) ausgebildet sind, die ihrerseits einen atmungsaktiven dehnbaren Kunststofffilm (25) umfasst, der mit einer Vielzahl von Luftdurchgängen (31) ausgebildet ist, die sich durch den Kunststofffilm (25) hindurch erstrecken und die jeweils eine erste Öffnung (32), die zur Haut des Trägers hinweist, und eine zweite Öffnung (33), die von der Haut des Trägers wegweist, aufweisen;
ein Paar atmungsaktiver, heißsiegelbarer faseriger Vliesstoffschichten (26), die mit beiden Oberflächen des Kunststofffilms an heißgesiegelten Punkten (27, 35) teilverschweißt sind, wobei die Windel (1A, 1 B) **dadurch gekennzeichnet ist, dass**:
die faserigen Vliesstoffschichten (26) mit einer Vielzahl von Raffungen (28) ausgebildet ist, die entlang Oberflächen der Verbundstoffbahn (18) ansteigen und abfallen;
die Anzahl der Luftdurchgänge (31), die durch den Kunststofffilm (25) ausgebildet sind, in einem Bereich von 10 bis 80 pro Quadratzentimeter des Kunststofffilms (25) liegt und insgesamt ein Flächenanteil der ersten Öffnungen (33) im Bereich von 10 bis 20 Prozent des Kunststofffilms (25) liegt;
die faserigen Vliesstoffschichten (26) innerhalb des Luftdurchgangs (31) an den heißgesiegelten Punkten (35) verschweißt sind und die Anzahl der Luftdurchgänge (31), innerhalb der die faserigen Vliesstoffschichten (26) miteinander verschweißt sind, im Bereich von 1,5 bis 10 % liegt; und
jeder der Flügel (14), die aus der elastisch dehnbaren Verbundstoffbahn (18) gebildet sind, eine Querdehnbelastung von 1,5 bis 7,0 N bietet, wenn der Flügel (14) in der Querrichtung auf eine Längenabmessung in der Querrichtung gedehnt wird, die 120% ihrer nicht gedehnten Querrichtungsabmessung entspricht, die als 100% gesetzt ist, und eine Querdehnbelastung von 3,0 bis 10,0 N bietet, wenn der Flügel (14) auf eine Längenabmessung in der Querrichtung gedehnt wird, die 150% der nicht gedehnten Längenabmessung entspricht.

2. Windel (1A, 1 B) nach Anspruch 1, wobei der Kunststofffilm (29) aus einem elastisch dehnbaren thermoplastischen Elastomerfilm (29) und einem Paar Polyolefin-basierten thermoplastischen Kunstharzfilmschichten (39) besteht, die auf beide Oberflächen des Elastomerfilms (39) geklebt sind, und wobei der faserige Vliesstoff (26) aus Polyolefin-basierten thermoplastischen Kunstharzfasern gebildet ist.

3. Windel (1A, 1 B) nach Anspruch 1 oder 2, wobei die zweite Öffnung (34) eine Fläche hat, die kleiner als diejenige der ersten Öffnung (33) ist, so dass sich der Luftdurchgang (32) von der ersten Öffnung (33) zur zweiten Öffnung (34) hin verjüngt.

4. Windel (1A, 1 B) nach einem der Ansprüche 1 bis 3, wobei die faserigen Vliesstoffschichten (26) an den heißgesiegelten Punkten (27, 35) einen faserigen Zustand beibehalten und einer Faserdichte aufweisen, die an den heißgesiegelten Punkten (27, 35) höher als in der verbleibenden Fläche ist.

5. Windel (1 A, 1 B) nach einem der Ansprüche 1 bis 4, wobei der Kunststofffilm (25) eine Dicke von 0,3 bis 1 mm hat und die faserigen Vliesstoffschichten (26) jeweils ein Flächengewicht von 20 bis 60 g/m², eine Dicke von 0,1 bis 0,5 mm und eine Dichte von 0,04 bis 0,6 g/cm³ haben.

## Revendications

1. Couche jetable (1A, 1 B), comprenant :
une région de taille avant (8) ;
une région de taille arrière (10) ;
une région d'entrejambe (9) s'étendant entre ces deux régions de taille (8, 10) ;
une paire de premières ailes (13) s'étendant vers l'extérieur à partir des parties de bord latéral (13a) transversalement opposées de ladite région de taille avant (8) dans une direction transversale ;
une paire de secondes ailes (14) s'étendant vers l'extérieur à partir des parties de bord latéral (14a) transversalement opposées de ladite région de taille arrière (10) dans la direction transversale ;
parmi lesdites premières et secondes ailes (13, 14), au moins lesdites secondes ailes (14) sont formées à partir d'une feuille composite (18) élastiquement étirable qui comprend, à son tour, un film plastique (25) extensible aéré formé avec une pluralité de passages d'air (31) s'étendant à travers ledit film plastique (25), ayant chacun une première ouverture (32) orientée vers la peau de l'utilisateur et une seconde ouverture (33) orientée à distance de la peau de l'utilisateur ;
une paire de couches de tissu non tissé fibreux thermosoudable aéré (26) partiellement collées sur les deux surfaces dudit film plastique à des points thermosoudés (27, 35), ladite couche (1A, 1 B) étant **caractérisée en ce que** :
lesdites couches de tissu non tissé fibreux (26) sont formées avec une pluralité de fronces (28) montant et descendant le long des surfaces de ladite feuille composite (18) ;
le nombre desdits passages d'air (31) formés à travers ledit film plastique (25) est dans une plage de 10 - 80/cm² dudit film plastique (25) et un rapport de surface totale desdites premières ouvertures (33) est de l'ordre de 10 - 20% du film plastique (25) ;
à l'intérieur dudit passage d'air (31), lesdites couches de tissu non tissé fibreux (26) sont collées ensemble au niveau desdits points thermosoudés (35) et le nombre desdits passages d'air (31) à l'intérieur desquels lesdites couches de tissu non tissé fibreux (26) sont collées entre elles est de l'ordre de 1,5 - 10% ; et
chacune desdites ailes (14) formées à partir de ladite feuille composite (18) élastiquement étirable présente une tension d'étirement transversale de 1,5 - 7,0 N lorsque ladite aile (14) est étirée dans la direction transversale par rapport à une dimension de longueur dans la direction transversale correspondant à 120% de sa dimension de longueur transversale non étirée déterminée comme étant de 100% et présente une tension d'étirement transversale de 3,0 - 10,0 N lorsque ladite aile (14) est étirée par rapport à une dimension de longueur dans la direction transversale correspondant à 150 % de ladite dimension de longueur non étirée.

2. Couche (1A, 1B) selon la revendication 1, dans laquelle ledit film plastique (29) est formé à partir d'un film élastomère thermoplastique (29) élastiquement étirable et d'une paire de couches de film en résine synthétique thermoplastique à base de polyoléfine (29) collées sur les deux surfaces dudit film élastomère (39) et dans laquelle ledit tissu non tissé fibreux (26) est formé à partir de fibres de résine synthétique thermoplastique à base de polyoléfine.

3. Couche (1A, 1 B) selon la revendication 1 ou 2, dans laquelle ladite seconde ouverture (34) a une surface inférieure à celle de ladite première ouverture (33) de sorte que ledit passage d'air (32) est progressivement rétréci à partir de ladite première ouverture (33) vers ladite seconde ouverture (34).

4. Couche (1A, 1B) selon l'une quelconque des revendications 1 à 3, dans laquelle lesdites couches de tissu non tissé fibreux (26) maintiennent un état fibreux au niveau desdits points thermosoudés (27, 35) et ont une densité fibreuse supérieure au niveau desdits points thermosoudés (27, 35) que dans la région restante.

5. Couche (1A, 1B) selon l'une quelconque des revendications 1 à 4, dans laquelle ledit film plastique (25) a une épaisseur de 0,3 - 1 mm, et lesdites couches de tissu non tissé fibreux (26) ont chacune un poids de base de 20 - 60 g/m², une épaisseur de 0,1 - 0,5 mm et une densité de 0,04 - 0,6 g/cm³.
